Europäisches Patentamt

European Patent Office

Office européen des brevets

(19)

(11) Publication number: **0 244 207 B1**

## EUROPEAN PATENT SPECIFICATION

(12)

(45) Date of publication of patent specification: **31.07.91**

(51) Int. Cl.⁵: **G01N 33/561**, G01N 33/537, //C12Q1/68

(21) Application number: **87303763.4**

(22) Date of filing: **28.04.87**

(54) Detection method and apparatus.

(30) Priority: **30.04.86 JP 97877/86**

(43) Date of publication of application:
**04.11.87 Bulletin 87/45**

(45) Publication of the grant of the patent:
**31.07.91 Bulletin 91/31**

(84) Designated Contracting States:
**DE FR GB IT NL**

(56) References cited:
**EP-A- 0 131 934**
**EP-A- 0 171 072**
**WO-A-84/03520**
**DE-B- 2 250 086**

(73) Proprietor: **TORAY INDUSTRIES, INC.**
**2, Nihonbashi-Muromachi 2-chome Chuo-ku**
**Tokyo 103(JP)**

(72) Inventor: **Murao, Yasuo**
**1-31-17 Tsunishi**
**Kamakura-shi Kanagawa-ken(JP)**
Inventor: **Hosaka, Shuntaro**
**3865 Jindaiji**
**Mitaka-shi Tokyo(JP)**

(74) Representative: **Ritter, Stephen David et al**
**Mathys & Squire 10 Fleet Street**
**London EC4Y 1AY(GB)**

## Description

### FIELD OF THE INVENTION

The present invention relates to a method and an apparatus for qualitatively or quantitatively detecting a test substance capable of forming a complex e.g. DNA, RNA or an immunological substance such as an antigen or antibody.

### DESCRIPTION OF THE PRIOR ART

Electrophoresis is widely used in separating DNA or RNA or in separating a variety of substances such as proteins, and involves separation by the use of differences in mobility of the substance in an electric field (see, e.g. DE-B2-2250086). This is done by using the fact that there are differences in size and electric charge among DNA, RNA and proteins. The separation is feasible in various areas because different mobilities are produced by adjusting concentration of the gel component of a buffer-containing polyacrylamide or agarose gel which is a generally applied medium in the electric field as well as by adjusting pH of the buffer solution. Then, such a separated substance is usually detected as bands on a considerably long area on the gel of plate form.

In nature, the nucleotide sequence of DNA or RNA is inherent to the species of viruses and organisms. The single stranded DNA or RNA forms a double strand (hybrid) specifically with single stranded DNA or RNA complementary to the former. By using this feature reagents for detecting DNA or RNA have been developed and are called DNA probes, Several kinds of DNA probe kits are available on the market. In detecting DNA or RNA by utilizing these reagents or kits, materials adsorbing and fixing DNA or RNA such as porous membranes of nitrocellulose or nylon are often employed in practical use. First, DNA or RNA to be detected are adsorbed on the above-mentioned adsorbent-fixer material by spotting a test solution on or passing it through and fixed by heating. The adsorbent-fixer material is treated with a DNA not complementary to the DNA or RNA to be detected, for example, salmon sperm DNA. Then a hybrid is formed between the labelled or labelable DNA which is complementary to the DNA or RNA to be detected, namely, DNA probe and the fixed DNA or RNA to be detected. After washing, the labelled substance is detected (when the probe has been labelled). Alternatively, if the probe has not been labelled, detection is made following treatment with a labeling agent and washing. In this case, if the labeling agent is a protein such as an enzyme, the residual adsorbing ability of the adsorbent-fixer material is blocked with a protein different from the labeling agent, for example, bovine serum albumin preceding the treatment with a labeling agent.

On the other hand, the general method for detecting an immunological substance, that is, for detecting an antibody in a specimen involves first placing a solution of an antibody against the antigen to be detected in a vessel made of a material highly adsorptive for proteins such as polystyrene to adsorb the antibody on the surface of the vessel at the wall and bottom, blocking the remaining adsorption capability of the vessel surface by treatment with an inactive protein such as bovine serum albumin, and after washing, placing a test solution in the vessel to contact antigen in the test solution with the adsorbed antibody thereby trapping the antigen by means of an antigen-antibody reaction. Then, a treatment is made with an antibody labelled with a radioisotope, a fluorescent agent or an enzyme to effect labeling by an antigen-antibody reaction followed by washing and then detection. In such detection method, in general, there is employed as the reaction vessel a plate with 96 wells.

There is often employed in the prior-art detection methods a support such as an adsorptive membrane, plastic or the wall of a vessel made of such a material (see e.g. "Rapid and sensitive colorimetric method for visualizing biotin-labeled DNA probes hybridized to DNA or RNA immobilized on nitrocellulose: Bio-blots"; Leary, J.J. et al Proc Natl Acad. Sci. USA Vol. 80 pp. 4045-4049, July 1983). Accordingly, the better the adsorption efficiency of a substance the greater will be the non-specific adsorption of the labelled substance. In order to reduce such non-specific adsorption, blocking of the portion in which the non-specific adsorption is possible by means of an inactive substance is required, and moreover, there is a need for carrying out thorough washing at each of the treatment stages. Therefore, the procedures become complicated, and a longer period of time is required.

### SUMMARY OF THE INVENTION

The objects of the present invention are to overcome such disadvantages of the prior-art methods to provide a detection method which is associated with a lower degree non-specific adsorption, is simpler in procedure and requires a shorter period of time and an apparatus used therefor.

According to the present invention, there is provided a method of detecting a test substance which comprises contacting the test substance with a labelled or labelable substance and detecting a complex formed between the test substance and the labelled or labelable substance, characterised in that uncomplexed labelled or labelable substance is separated by electrophoresis on a gel which is chosen such that free labelled or labelable substance, when subjected to electrophoresis, will pass through and that the labelled or labelable substance which forms a complex with the test substance will not, the separation by electrophoresis being carried out prior to detection of said complex.

The invention also provides apparatus for carrying out the above method which comprises a plurality of tubular passages which are each open at one end thereof and closed with a gel layer at the other end for providing a space to maintain a specimen containing the reaction mixture of the test substance and a labelled or labelable substance, the tubular passages being so arranged as to allow electrophoresis of the specimen through the gel.

Test substances as used herein include DNA or RNA of virus-, microorganism-, animal- or plant-origin, or immunological substances such as antigen or antibody. Labelled substance as used herein refers to DNA or RNA that is complementary to the DNA or RNA of virus-, microorganism-, animal- or plant-origin, and immunological substances to which a labeling agent such as a fluorescent, chemiluminescent, radioisotopic or enzymatic agent is attached. The labelled substance of the present invention may be either one to which a labeling agent is directly attached as described above, or one in which the DNA, RNA or immunological substance is attached via a ligand or hapten such as biotin, a dinitrophenyl group or a sulfonyl group to an antibody against a hapten or avidin to which a labeling agent is attached.

One of the characteristic features of the present invention lies in the feasibility of treating the specimen solution containing a test substance with a labelled substance in a homogeneous system within a vessel. Another feature is the use of electrophoresis as a means of detecting the test substance by measuring the labelled substance which forms a complex remaining from separation following removal of the labelled substance which does not form the complex, these two labelled substances having been coexistent in a system.

## DESCRIPTION OF THE DRAWINGS

Fig.1 shows an apparatus according to an embodiment of the present invention, in which (a) is a sectional view of the apparatus during electrophoresis and (b) is a plan view thereof;
Fig.2 shows another example of a hole shape in the apparatus;
Fig.3 shows a detecting apparatus according to another embodiment of the present invention, having a tubular passage constituted by tubes;

and
Fig.4 shows an apparatus according to a further embodiment of the present invention, using a gel molding having a plurality of wells, in which (a) is a perspective view and (b) is a sectional view.

In the drawings:
1       plate
2       hole
3       gel layer
4       specimen layer
5       electroconductive solution
6, 6'   electrodes opposite in polarity
7       tube
8       well
9       gel molding

## DESCRIPTION OF THE PREFERRED EMBODIMENTS

An embodiment of the means for separating by electrophoresis the labelled substance that does not form the complex to remove it may be illustrated by the methods set forth below. One of these is subjecting to electrophoresis a specimen of the test substance treated with a labelled substance by being applied as a layer on to a gel layer. Thickness of the gel layer may be appropriately chosen, but usually, it is set in a range between 1 and 3 mm in consideration of conditions such as time of electrophoresis and mechanical strength. The gel layer is desirably made of a material such as agarose or polyacrylamide fixed at a concentration through which the complex does not, or only to a small extent, passes and through which the labelled substance easily passes. These arrangements allow the electrophoresis to proceed from the specimen layer toward the gel layer to maintain the complex on the surface of or within the gel layer and to pass the labelled substance that does not form the complex through the gel layer. In order to prevent convection of the specimen layer, the layer is preferably made to gel by the addition of a material such as agarose at such a concentration that the complex and the labelled substance can move. It may be arranged, however, to allow the labelled substance that does not form a complex to move in a direction opposite to the complex by adjusting the pH of the electrophoretic medium.

An alternative method is one in which the specimen layer is incorporated within the gel layer. It is arranged in such a way that the specimen solution is incorporated into a gel layer of a material such as agarose or polyacrylamide at a concentration through which the complex does not, or only to a small extent, passes, but through which the labelled substance easily passes, to remove the labelled substance that does not form the complex from the gel layer.

Then, in these cases, it is essential that the complex is large enough as compared with the labelled substance to facilitate the separation. In the case where the test substance is e.g. DNA or RNA, the substance is in nature large enough to allow satisfactory separation by employing labelled DNA, complementary to the former, that is short in nature or has been cut short by such a means as an enzyme or ultrasonic treatment. On the other hand, if the test substance is e.g. an antigen or antibody which is of approximately the same size as that of the labelled substance, separation will become feasible by forming a complex of the labelled substance with the test substance bound to a substance significantly larger than the labelled substance such as, for example, a polymer of antibody against the antigen or antigen against the antibody or a polymer to which the antigen or antibody is bound.

The apparatus of the present invention, which is for detecting a test substance, may include tubular passages which are each open at one end thereof and closed with a gel layer at the other end for providing a space to maintain and migrate a specimen containing the reaction mixture of the test substance and a labelled substance and to be subjected to electrophoresis, the apparatus may further include means for subjecting the specimen to electrophoresis.

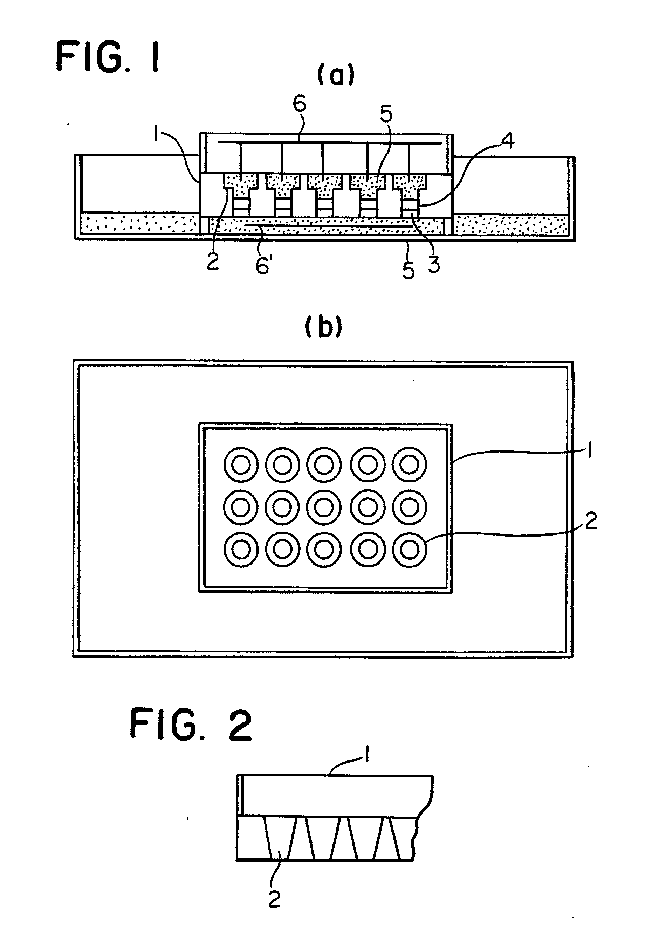

The apparatus shown in Fig.1 is provided with a plate 1 with a large number of through holes 2 to maintain the specimen or the gel, and a conductive solution layer 5 to subject the specimen layer 4 and the gel layer 3 to electrophoresis. The conductive solution layer is composed of two liquid layers each of which contains the mutually different electrode 6 or 6'. As shown in Fig.1, the conductive liquid layer 5 may be arranged in such a way that one of the liquid layer is within each of the holes 2. Alternatively, it may be on top of the holes. Any form may be used provided the two conductive solution layers are arranged on opposite sides of the specimen layer 4 and the gel layer 3 to allow for electrophoresis to occur in both layers.

Also, as shown in Fig.1, the mutually opposed electrodes 6 and 6' also may be arranged in such a way that one of the electrodes is set in the liquid layer of each of the holes as with 6, or alternatively, it may be set as with 6'. The plate 1 is desirably set horizontally. However, a vertical or diagonal arrangement may be used. The plate may be made of any material. Examples include plastics such as polystyrene, polycarbonate, polyethylene and polymethyl methacrylate, glass, gels such as agarose and polyacrylamide.

In Fig.1, a plurality of tubular passages which are each open at one end thereof and closed with a gel layer at the other end, is consituted by a plate 1 which has a plurality of through holes 2. The shape of the holes 2 is not specially limited, but a circular shape is preferred, and any sectional shape may be adopted. For example, those holes may be of such a shape as shown in Fig. 1, or may be tapered as in Fig. 2. Preferably, the holes are 3 to 5 mm in diameter and 1 to 2 cm in length and it is preferred that the holes are arranged regularly.

Ideally, plate 1 has a shape similar to the 96-well plate generally used in an immunological detecting method.

The gel layer may be used in such a mode as shown in Fig.1 in which it is held within the holes and a specimen layer is placed thereon. Alternatively, the gel layer may be used in such a mode that it is present outside the holes in close contact with the lower ends of the holes and a specimen layer is placed thereon through the holes. By so doing a tubular passage sealed by the gel layer is formed.

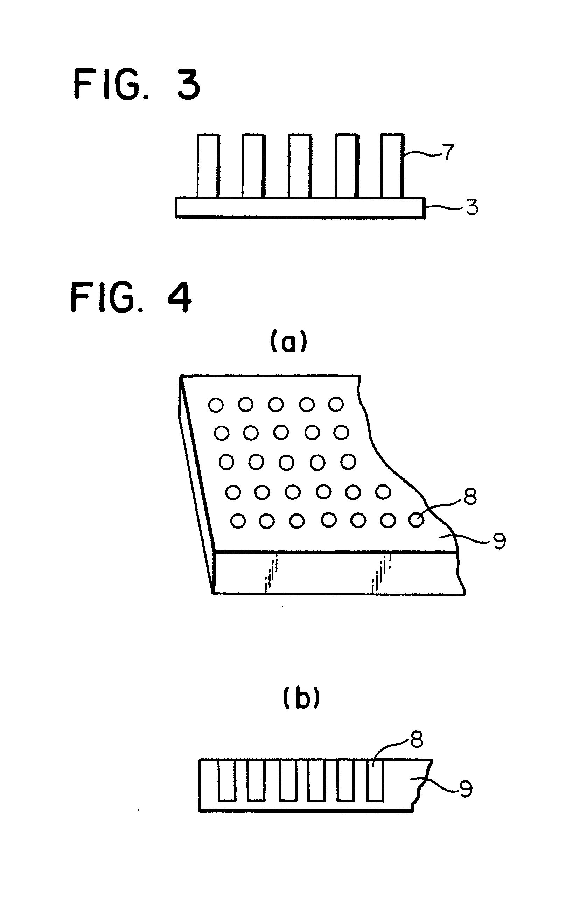

As the tubular passage, in addition to the plate having a plurality of holes as referred to above, there also may be adopted tubes 7 of a cylindrical or like shape formed of a plastic material, for example as shown in Fig. 3. In this case, the gel layer 3 may be brought into close contact with one ends of the tubes 7 as shown in Fig.3, or one ends of the tubes 7 may be lightly pressed in to the gel layer 3. Of course, it is possible to hold the gel layer 3 in the tubes 7 and place a specimen layer thereon, as shown in Fig. 1.

In the apparatus of the present invention, moreover, the tubular passages and the gel layer may be integrated into a gel molding 9 having a plurality of wells 8, as shown in Fig.4.

The detection method according to the present invention will be described below. First, each of the specimens is placed in a reaction vessel to which is then added a labelled substance. The mixture reacts to form a complex. Separately, a gel layer is prepared in the holes of the plate as shown in Fig. 1. The gel layer may have been prepared in advance. As shown in Fig.1, the reaction solution is applied as a layer on to the gel layer, or preferably, the reaction solution containing the gel component added at such concentration as described above is similarly applied as a layer on the gel layer. Subsequently, the plate is set in such a way that the lower surface of the plate is in contact with the surface of the conductive solution layer. Then, a conductive solution is poured on to the upper surface of the plate to form the upper conductive solution layer, followed by application of electric current at a predetermined voltage for a predetermined period of time. Then, the labelled substance is measured. It is preferable to do this while maintaining the gel layer in the holes.

Conventional detection methods are used. For example, where the labelling agent is enzyme, the detection can be done by color development of the labelling enzyme through substrate. Where the labelling agent is a radioisotope, the use of a scintillation counter or a Geiger counter permits detection, or exposure of film to light is effective. Where biotin or hapten is used as the labelled substance of the complex, a further reaction is allowed to take place between the complex and avidin or antibody with a labelling agent such as enzyme immobilized thereto, and thereafter the labelling agent is measured according to a conventional method, whereby the detection can be effected.

In the case where the gel layer is formed outside the holes 2 in the apparatus of Fig.1, or where the gel layer is provided at one end of the tubes 7, as in Fig. 3, only, specimen may be placed in the holes 2 or the tubes 7. Also in the case of the gel molding shown in Fig.4, only specimen may be placed in the wells 8 because the bottom of the gel molding 9 corresponds to the gel layer. The following operations are the same as in the case of the plate shown in Fig.1.

In the detection method according to the present invention, it is feasible to form a complex of the test substance with a labelled substance by treatment in a homogeneous system in a single vessel. The treatment step for inhibiting non-specific adsorption and the washing step required in the prior-art methods may be omitted. Time requirement can also be reduced. Furthermore, there is no clogging of the porous membrane of nitrocellulose or nylon associated with the filtration-adsorption method described above so that treatment of a large amount of test material is feasible.

Examples are given below to describe the present invention in more details.

Example 1

(1) Preparation of biotin-labelled φX-174RF DNA

To a mixture of 5 μl of solution A4 of Nick translation reagent (BRL, Gaitherburg, Maryland 20877, U.S.A.) (each 0.2 mM dATP, dCTP and dGTP), 2 μl of φX-174RF DNA solution (0.5 μg/μl), 2.5 μl of 0.4 mM biotin-11-dUTP and 35.5 μl of solution E ($H_2O$) was added 5 μl of solution C (0.4 U/μl DNA polymerase, 40 pg/μl deoxyribonuclease). The resulting mixture was incubated at 15°C for 1.5 hours. To the reaction mixture were added 5 μl of the solution D (300 mM EDTA) and 1.25 μl of 5% aqueous solution of SDS. The resulting mixture was introduced into 5 ml of Sephadex® G-50 column, which was eluted with 1XSSC (0.15 M NaCl, 15 mM sodium citrate, pH = 7.0). Each 150 μl of the eluates was collected. Each of the eluates was spotted in an amount of 2 μl on a nitrocellulose filtration membrane. The membrane was heated at 80°C for 30 minutes. The filtration membrane was soaked in a blocking buffer [PBS (0. 13 M NaCl, 7 mM $Na_2HPO_4$, 3 mM $NaH_2PO_4$) containing 2% BSA, 0.05% Triton® X-100 and 5 mM EDTA] at room temperature for 30 minutes. It was then soaked in a solution of Detek-1-acp detection complex (ENZO, 325 Hudson Street, New York, N.Y. 10013, U.S.A.) diluted 1:200 with its dilution buffer. It was then washed five times with a washing buffer (0.5 M NaCl, 0.5% Triton® X-100, 1 mM EDTA, 2% BSA, 10 mM $KPO_4$, pH = 6.5) each for 5 minutes and twice with a predetection buffer (0.2 M sodium acetate, pH = 5.8) each for 2 minutes. The filtration membrane was soaked in a 100:1 mixture of 1 mM naphthol AS-MX phosphate and Fast Violet B salt 4 mg/ml followed by incubation at room temperature for 15 minutes. Eluate fractions with color developed was collected to obtain ca. 1 μg/ml solution of biotin-labelled φX-174RF DNA.

(2) Detection of biotin-labelled φX-174RF DNA.

The solution of biotin-labelled φX-174RF DNA obtained under (1) above was dialyzed overnight against 1XTBE (89 mM Tris base, 89 mM boric acid, 2 mM EDTA, PH = 8.0) to form 1 μg/ml 1XTBE solution of the biotin-labelled φX-174RF DNA. The solution was diluted with 1XTBE to five specimens. 1 μg/ml, 100 ng/ml, 10 ng/ml and 1XTBE only. Each 50 μl of the specimens was placed in a sample tube, to which 5 μl of 1:20 diluted solution of a detection complex of Detek-1-acp (ENZO). The mixture was incubated at 42°C for 10 minutes. Separately, 50 μl of a heated solution of 1XTBE containing 3% agarose (BRL.) was introduced into the holes of a multi-hole plate with one side blocked by an adhesive tape of the electrophoresis apparatus shown in Fig.1 and cooled to form a gel. Separately, to the test solution was added each 50 μl of a heated soluticn of 1XTBE containing 1.2% agarose, and the mixture was thoroughly stirred. Each 50 μl of the mixture was applied as a layer on to the gel layer which had been prepared in advance, and allowed to form a gel. The adhesive tape of the multi-hole plate was removed to allow for such an arrangement that the plate was in contact with the surface of the 1XTBE solution placed in the conductive solution layer. After introducing 1XTBE also in to the conductive solution of the multi-hole plate, electric current was applied at a voltage of 70V for 15 minutes using the electrode of the multi-hole plate as the cathode. The gel layer was then soaked in a 100:1 mixture of 1 mM naphthol AS-MX phosphate and predetection buffer solution of Fast Violet B salt 4 mg/ml at room temperature for 15 hours to develop color. The color development was observed on the intersurface of the gel layer and the specimen layer. The lower limit of color development was 1 ng/ml of biotin-labelled $\phi$X-174RF DNA in the solution.

Example 2

(1) Cutting of biotin-labelled $\phi$X-174RF DNA

A 1 $\mu$g/ml 1XSSC (0.15 M NaCl, 15 mM sodium citrate, pH = 7.0) of biotin-labelled $\phi$X-174RF DNA was treated with an ultrasonic breaker (Kaijo Denki 4280 (six time at 1A for 10 seconds)).

(2) Detection of $\phi$X-174 single-stranded DNA

Each 25 $\mu$l of 1 $\mu$g/ml, 100 ng/ml, 10 ng/ml and 1 ng/ml 1XSSC solution of $\phi$X-174 single-stranded DNA (BRL) was placed in a sample tube, to which was added each 25 $\mu$l of the ultrasonic-treated biotin-labelled $\phi$X-174RF DNA (1 $\mu$g/ml). The mixture was boiled for 5 minutes and then incubated at 60°C for 30 minutes and subsequently at 42°C for 10 minutes. To the incubated mixture was added 5 $\mu$l of 1:20 diluted solution of a detection complex of ENZO Detek-1-acp followed by incubation at 42°C for 1 minute. To the incubated mixtures was added each 50 $\mu$l of a heated solution of 1XTBE containing 1.2% agarose at 42°C, and the mixture was thoroughly stirred. As in Example 1, 50 $\mu$l of the mixture was applied as a layer on to a 3% agarose gel layer (prepared in advance) to form a gel. Electric current was applied at 70V for 20 minutes in the same way as in Example 1. The gel layer was soaked in a 100:1 mixture of 1 mM naphthol AS-MS phosphate and a predetection buffer solution of Fast Violet B salt at room temperature for 15 hours. Color development was observed. The detection limit of $\phi$X-174 single-stranded DNA in the solution was 1 ng/ml.

## Claims

1. A method of detecting a test substance which comprise contacting the test substance with a labelled or labelable substance and detecting a complex formed between the test substance and the labelled or labelable substance, characterised in that uncomplexed labelled or labelable substance is separated by electrophoresis on a gel which is chosen such that free labelled or labelable substance, when subjected to electrophoresis, will pass through and that the labelled or labelable substance which forms a complex with the test substance will not, the separation by electrophoresis being carried out prior to detection of said complex.

2. An apparatus for carrying out the method claimed in Claim 1 which comprises a plurality of tubular passages (2) which are each open at one end thereof and sealed with a gel layer (3) at the other end for providing a space (4) to maintain a specimen containing the reaction mixture of the test substance and a labelled or labelable substances, the tubular passages being so arranged as to allow electrophoresis of the specimen through the gel.

3. An apparatus according to Claim 2 wherein said tubular passages are constituted by a plate (1) having a plurality of holes (2).

4. An apparatus according to Claim 2, wherein said tubular passages sealed with a gel layer comprise a gel molding (9) having a plurality of wells (8).

## Revendications

1. Procédé pour détecter une substance d'essai qui comprend les étapes consistant à mettre en contact la substance d'essai avec une substance marquée ou pouvant être marquée et a détecter un complexe formé entre la substance d'essai et la substance marquée ou pouvant être marquée, caractérisé en ce que la substance marquée ou pouvant être marquée, non complexée, est séparée par électrophorèse sur un gel qui est choisi de façon que la substance marquée ou non marquée, libre, lorsqu'elle est soumise à l'électrophorèse, traverse et que la substance marquée ou pouvant être marquée qui constitue un complexe avec la substance d'essai ne passe pas, la séparation par électrophorèse étant exécutée avant la détection du complexe.

2. Dispositif pour exécuter le procédé de la revendication 1, qui comprend une multitude de passages tubulaires (2) qui sont chacun ouverts à l'une de leurs extrémités et fermés de manière étanche avec une couche de gel (3) à l'autre extrémité afin de fournir un espace (4) pour maintenir un échantillon contenant le mélange réactionnel de la substance d'essai et une substance marquée ou pouvant être marquée, les passages tubulaires étant agencés de façon à permettre l'électrophorèse de l'échantillon à travers le gel.

3. Dispositif selon la revendication 2, dans lequel les passages tubulaires sont constitués par une plaque (1) présentant une multitude de trous (2).

4. Dispositif selon la revendication 2, dans lequel les passages tubulaires rendus étanches avec une couche de gel comprennent un moulage de gel (9) ayant une multitude de puits (8).

**Patentansprüche**

1. Verfahren zum Nachweis einer Testsubstanz, bei dem man die Testsubstanz mit einer markierten oder markierbaren Substanz in Kontakt bringt und einen zwischen der Testsubstanz und der markierten oder markierbaren Substanz gebildeten Komplex nachweist, dadurch gekennzeichnet, daß man die nicht-komplexierte markierte oder markierbare Substanz durch Elektrophorese in einem Gel abtrennt, das so gewählt wird, daß die freie markierte oder markierbare Substanz, wenn sie einer Elektrophorese ausgesetzt wird, es durchläuft und daß die markierte oder markierbare Substanz, die einen Komplex mit der Testsubstanz bildet, es nicht durchläuft, wobei die Trennung durch die Elektrophorese vor dem Nachweis des Komplexes durchgeführt wird.

2. Vorrichtung zur Durchführung des Verfahrens nach Anspruch 1 umfassend eine Vielzahl von röhrenförmigen Durchgängen (2), die an einem Ende offen sind und am anderen Ende mit einer Gelschicht (3) verschlossen sind, um einen Raum (4) auszubilden, um eine Probe aufzunehmen, die die Reaktionsmischung der Testsubstanz und einer markierten oder markierbaren Substanz enthält, wobei die röhrenförmigen Durchgänge so angeordnet sind, daß sie die Elektrophorese der Probe durch das Gel erlauben.

3. Vorrichtung nach Anspruch 2, in der die röhrenförmigen Durchgänge von einer Platte (1) mit einer Vielzahl von Löchern (2) gebildet werden.

4. Vorrichtung nach Anspruch 2, in der die mit einer Gelschicht verschlossenen röhrenförmigen Durchgänge ein Gel-Formteil (9) mit einer Vielzahl von Vertiefungen (8) umfassen.

# FIG. 1

## (a)

## (b)

# FIG. 2

# FIG. 3

# FIG. 4

## (a)

## (b)